# EUROPEAN PATENT SPECIFICATION

(11) **EP 2 136 206 B1**
(45) Date of publication and mention of the grant of the patent: **11.04.2012**
(21) Application number: 08711870.9
(22) Date of filing: 24.02.2008
(51) Int. Cl.: C12Q 1/00, C12Q 1/28

(54) **METHOD FOR DETERMINING GLUCOSE CONCENTRATION**
VERFAHREN ZUR GLUCOSEKONZENTRATIONSBESTIMMUNG
PROCÉDÉ POUR DÉTERMINER LA CONCENTRATION DE GLUCOSE

(30) Priority: 24.02.2007 JP 2007044771
(43) Date of publication of application: 23.12.2009
(73) Proprietor: ARKRAY, Inc., Kyoto-shi, Kyoto 601-8045 (JP)
(72) Inventor: KAMATA, Tatsuo, Kyoto-shi Kyoto 601-8045 (JP); TAKAGI, Takeshi, Kyoto-shi Kyoto 601-8045 (JP)
(74) Representative: Spilgies, Jan-Hendrik
(86) International application number: PCT/JP2008/053106
(87) International publication number: WO 2008/102889

(56) References cited:
- EP-A1- 2 083 274
- EP-A2- 1 130 111
- WO-A1-94/24262
- WO-A1-03/010529
- GB-A- 1 332 431
- JP-A- 01 129 153
- JP-A- 55 092 694
- SCHLAPFER P ET AL: "Electrochemical measurement of glucose using various electron acceptors" CLINICA CHIMICA ACTA, ELSEVIER BV, AMSTERDAM, NL, vol. 57, no. 3, 17 December 1974 (1974-12-17), pages 283-289, XP025198895 ISSN: 0009-8981 [retrieved on 1974-12-17]
- J. WANG: 'Analytical Electrochemistry', 2006, J. WILEY AND SONS, NEW JERSEY pages 1 - 28

## Description

### TECHNICAL FIELD

The present invention relates to a method for carrying out the measurement of the glucose concentration in a blood specimen.

### BACKGROUND ART

There is a conventionally known method called an enzyme-electrode method, which is a method for measuring a glucose concentration. According to the method, information correlating with a glucose concentration in a specimen is outputted to an electrode in contact with the specimen, and the glucose concentration is calculated based on the output. In an example of the method, electrons are removed at an electrode from hydrogen peroxide generated by oxidizing glucose with an enzyme, and a glucose concentration is calculated based on a current value measured at the time in the electrode (for example, see the Patent Document 1)

When the glucose concentration is measured, blood may be used as the specimen in some cases. In a case where hemolysis in hemocyte is generated, hemoglobin may be included in the specimen whether the whole blood is used or blood serum or plasma is used.

However, the hemoglobin easily reacts with the hydrogen peroxide. Therefore, the hemoglobin included in the specimen inhibits the reaction between the hydrogen peroxide and the electrode. As a result, the electrode output tends to show a lower value as the reaction between the hemoglobin and the hydrogen peroxide advances in the presence of the hemoglobin, which makes it difficult to obtain high measuring accuracy.

It is not avoidable for the specimen to include the hemoglobin by preventing the hemolytic activity in the hemocyte. This is, however, not a realistic approach because it is necessary to pay a meticulous attention during the transportation, preservation and pretreatment of the blood, which demands burdensome handling steps. Another disadvantage is the variability in easiness of the hemolytic activity depending on an analyte.

[Patent Document 1] JP-B-H07-37991

WO 94/24262 describes the immobilization of horseradish peroxidase on colloidal gold and subsequent deposition on an electrode surface so as to prepare biosensors for mediatorless glucose determination.

Schläpfer et al., Clinica Chimica Acta, 57 (1974) 283 - 289, describe the electrochemical measurement of glucose using various electron acceptors.

JP 55092694 describes the use of a buffer solution containing azide ions and nitrite ions to determine the blood sugar concentration in whole blood.

GB 1 332 431 describes a method of providing a measure of the change of the oxygen concentration in a blood sample.

EP 2 083 274, which constitutes prior art pursuant to Article 54 (3) EPC, relates to a blood analyzer for measuring concentrations of glucose and glycohemoglobin in blood.

US 2004/0106166 provides an enzyme electrode exhibiting good measurement performance under wide ranges of the application conditions.

JP 01129153 describes a method of measuring the content of amylose in starch in a flow type measuring apparatus.

### DISCLOSURE OF THE INVENTION

### PROBLEM TO BE SOLVED BY THE INVENTION

An object of the present invention is to improve measurement accuracy by controlling any influences from an inhibitor when the concentration of glucose as a substrate in a blood specimen is measured without any complicated and time-consuming handling steps.

### MEANS FOR SOLVING THE PROBLEMS

According to a first aspect of the present invention, there is provided a method for measuring the concentration of glucose as a substrate in a blood specimen containing hemoglobin and glucose, based on the amount of hydrogen peroxide generated from the substrate, wherein a suppressing agent for suppressing a reaction between the hydrogen peroxide and hemoglobin as an inhibitor is allowed to coexist. The method of the present invention comprises the steps of contacting the blood specimen with a reagent comprising a diluent, an azide compound as a suppressing agent for suppressing the reaction between hydrogen peroxide and hemoglobin by transforming hemoglobin into methemoglobin, and sodium chloride or potassium chloride as a supporting electrolyte, wherein the concentration of the azide compound in the reagent is 0.001 to 1.000 wt%, and measuring the concentration of hydrogen peroxide generated from the glucose with an enzyme electrode 30 comprising a substrate selective transmission film 33, an enzyme immobilized film 34, a hydrogen peroxide selective transmission film 35, and a hydrogen peroxide electrode 36, wherein the substrate selective transmission film 33 is a film for selectively supplying glucose in the specimen to the enzyme immobilized film 34, and the hydrogen peroxide selective transmission film 35 is a film for selectively supplying hydrogen peroxide to the hydrogen peroxide electrode 36.

As the suppressing agent, an azide compound which transforms the hemoglobin as an inhibitor into methemoglobin is used.

Examples of the azide compound to be used are: sodium azide; 4-dodecylbenzenesulfonyl azide; 4-acetylaminobenzenesulfonyl azide; diphenylphosphoric azide; iron azide; hydrogen azide; lead azide; mercury azide; copper azide; and silver azide. The concentration of the azide compound in the measuring system is 0.001 to 1.000 wt%, and preferably 0.010 to 0.100 wt%.

In the present invention, a supporting electrolyte is further allowed to coexist (to be added). The supporting electrolyte to be used is sodium chloride or potassium chloride, and sodium chloride is preferably used as the supporting electrolyte. The concentration of the supporting electrolyte is preferably 0.010 to 2.000 wt%, and more preferably 0.050 to 0.500 wt%.

The substrate used in the method of the present invention is glucose. The substrate is included in a blood specimen.

According to the method of the present invention, an enzyme-electrode method is employed to measure the amount of the hydrogen peroxide.

### BEST MODE FOR CARRYING OUT THE INVENTION

The present invention is described below referring to the drawings.

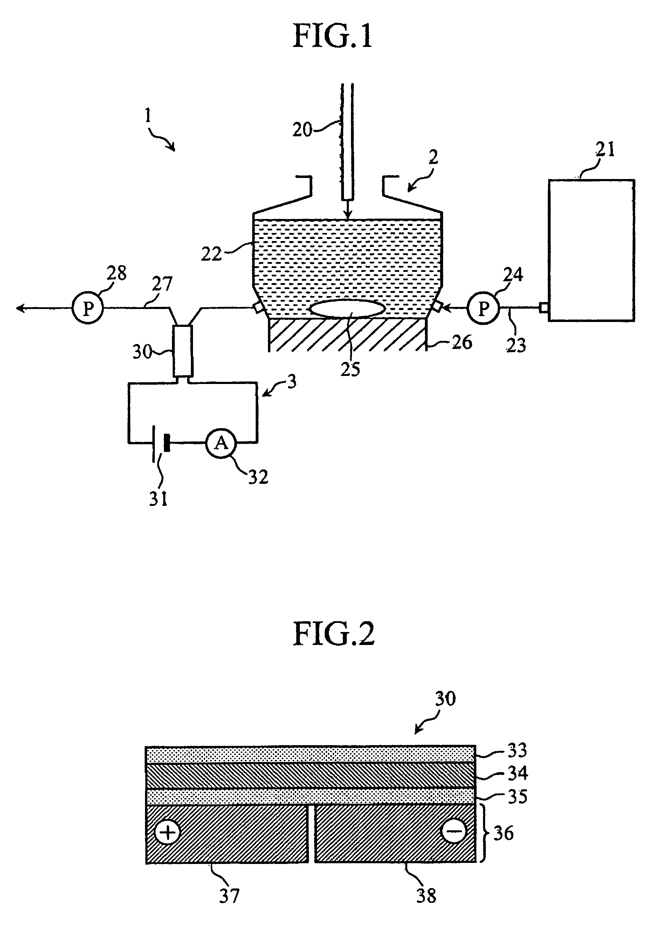

A concentration measuring device 1 illustrated in Fig. 1 is configured to measure a specimen adjusted in a specimen preparation mechanism 2 using a measurement mechanism 3.

The specimen preparation mechanism 2 is a mechanism for preparing a specimen from an analyte, and comprises a nozzle 20, a reagent bottle 21, and a diluting / mixing tank 22.

The nozzle 20 is a nozzle for supplying an analyte into the diluting / mixing tank 22. The analyte to be used is a blood specimen, or diluents obtained therefrom. Any of the whole blood and blood serum or plasma can be used.

The reagent bottle 21 retains therein a reagent for diluting an analyte or washing the diluting / mixing tank 22. The reagent bottle 21 is connected to the diluting / mixing tank 22 with a pipe 23 interposed therebetween. A pump 24 is provided at an intermediate position in the pipe 23, and a configuration is given such that a force generated by the pump 24 serves to supply the reagent retained in the reagent bottle 21 into the diluting / mixing tank 22.

A material including a diluent and a suppressing agent is used as the reagent.

An example of the diluent is a buffer solution. The buffer solution is not particularly limited as far as it can adjust the reaction pH of a substrate in a targeted range, and phosphates, for example, can be used. The concentration of the buffer solution in the reagent is set to, for example, 0.0001 to 0.1000 M.

The suppressing agent serves to suppress a reaction between hydrogen peroxide as the substrate and an inhibitor. The inhibitor is hemoglobin, and an azide compound which transforms the hemoglobin into methemoglobin is used as the suppressing agent.

Examples of the azide compound to be used are: sodium azide (NaN₃); sodium azide; 4-dodecylbenzenesulfonyl azide; 4-acetylaminobenzenesulfonyl azide, diphenylphosphoric azide; iron azide; hydrogen azide; lead azide; mercury azide; copper azide; and silver azide. The concentration of the azide compound included in the reagent is 0.001 to 1.000 wt%, preferably 0.010 to 0.100 wt%.

The reagent further includes a supporting electrolyte, which is sodium chloride or potassium chloride. The concentration of the supporting electrolyte in the reagent is, for example, 0.010 to 2.000 wt%, and preferably 0.050 to 0.500 wt%.

The diluting / mixing tank 22 is a tank for providing a place where an analyte is diluted so that a specimen is prepared. The diluting / reaction tank 22 is configured such that the analyte is supplied by means of the nozzle 20, and the reagent is supplied from the reagent bottle 21. The diluting / mixing tank 22 comprises therein an agitator 25. When the agitator 25 is rotated by a stirrer 26, the analyte and the reagent are mixed with each other. The diluting / mixing tank 22 is further connected to an enzyme electrode 30 of the measurement mechanism 3 with a pipe 27 interposed therebetween. A pump 28 is provided at an intermediate position in the pipe 27, and a configuration is given such that a force generated by the pump 28 serves to supply a specimen prepared in the diluting / mixing tank 22 to the enzyme electrode 30 of the measurement mechanism 3.

The measurement mechanism 3 comprises the enzyme electrode 30, a power supply 31 and a current value measuring unit 32.

The enzyme electrode 30 outputs an electrophysical quantity corresponding to the amount of electrons transferred to and received from a substrate in a specimen. As illustrated in Fig. 2, the enzyme electrode 30 comprises a substrate selective transmission film 33, an enzyme immobilized film 34, a hydrogen peroxide selective transmission film 35, and a hydrogen peroxide electrode 36.

The substrate selective transmission film 33 is a film for selectively supplying glucose as a substrate in a specimen to the enzyme immobilized film 34. The substrate selective transmission film 33 to be used is a various conventionally available film, although being selected depending on the type of a substrate.

The enzyme immobilized film 34 a film for generating hydrogen peroxide by oxidizing a substrate, and is configured to include an oxidase. The oxidase used in the present invention is selected depending on the type of a substrate. The substrate to be measured by the concentration measuring device 1 is glucose, and glucose oxidase oxidase can be mentioned as the oxidase.

The hydrogen peroxide selective transmission film 35 is a film for selectively supplying hydrogen peroxide to the hydrogen peroxide electrode 36. Examples of the hydrogen peroxide selective transmission film 35 to be used are acetylcellulose-based or polyarylamine-based films.

The hydrogen peroxide electrode 36 outputs an electrical signal corresponding to the amount of the supplied hydrogen peroxide, in other words, the concentration of the substrate. The hydrogen peroxide electrode 36 to be used is an electrode in which platinum is used as an anode 37, while silver is used as a cathode 38.

The power supply 31 illustrated in Fig. 1 applies a voltage to the enzyme electrode 30 (hydrogen peroxide electrode 36). A direct-current power supply, for example, is used as the power supply 31, and the voltage applied to the enzyme electrode 30 (hydrogen peroxide electrode 36) is set to, for example, 0.1 to 1.0 V.

The current value measuring unit 32 is a unit for measuring the amount of electrons transferred to and from the hydrogen peroxide electrode 36 and the hydrogen peroxide as a current value. The current value is intermittently measured by the current value measuring unit 32, and a measurement interval is set to, for example, 50 to 200 msec.

Next is described an operation of the concentration measuring device 1 in a case where glucose in the whole blood is a target of the measurement.

When the concentration of the glucose in the whole blood is measured, the whole blood and the reagent are supplied into the diluting / mixing tank 22. The whole blood is supplied into the diluting / mixing tank 22 by means of the nozzle 20, and the amount of the whole blood to be supplied is set to, for example, 4 to 20 µL. On the other hand, the reagent is supplied into the diluting / mixing tank 22 by means of the pump 24, and the amount of the reagent to be supplied is set to, for example, approximately 100 times as much as the amount of the whole blood.

When the whole blood and the reagent are supplied in the concentration measuring device 1, they are agitated by the agitator 25 in the diluting / mixing tank 22. Because the amount of the reagent to be supplied is set to approximately 100 times as much as the amount of the whole blood to be supplied, the pH of the buffer solution in the diluting / mixing tank 22 and the concentrations of the suppressing agent and the supporting electrolyte are substantially retained to be equal to the pH and the concentrations in the reagent. After that, the prepared specimen is supplied to the enzyme electrode 30 by means of the pump 28.

In the enzyme electrode 30, the glucose transmits through the substrate selective transmission film 33 and is then supplied to the enzyme immobilized film 34. In the enzyme immobilized film 34, the glucose of the oxidase (glucose dehydrogenase) is oxidized, and gluconic acid and hydrogen peroxide are generated as illustrated in the following reaction formula (1). The hydrogen peroxide transmits through the hydrogen peroxide selective transmission film 35 and is then supplied to the hydrogen peroxide electrode 36. In the hydrogen peroxide electrode 36, the reaction represented by the following reaction formula (2) is generated by the application of the voltage by the power supply 31, and the hydrogen peroxide supplies electrons to the anode 37 to be decomposed into oxygen and hydrogen ions. At the time, the electrons supplied to the anode 37 generate a current flow between the anode 37 and the cathode 38, and the current generated at the time is measured by the current value measuring unit 32. On the other hand, in the cathode 38, the electrons from the anode 37 generate the reaction represented by the following reaction formula (3), whereby water is produced.

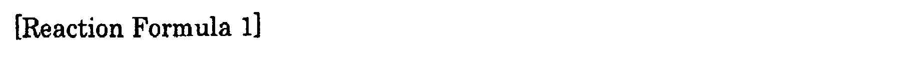

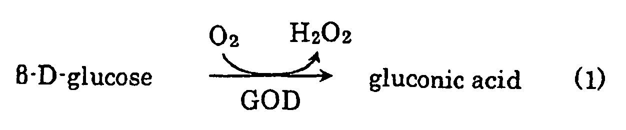

anode: H₂O₂ → 2H⁺ + O₂ + 2e⁻ (2)

cathode: 4H⁺ + O₂ + 4e⁻ → H₂O (3)

In the anode 37, the reductive reaction of the hydrogen peroxide (H2O2) is generated. In the presence of oxyhemoglobin (Fe (II)) in the diluting / mixing tank 22, the anodic reaction and the oxidizing reaction of deoxyhemoglobin (oxy-Hb (Fe II)) into methemoglobin (met-Hb (Fe (III)) compete with each other as illustrated in the following reaction formula (4).

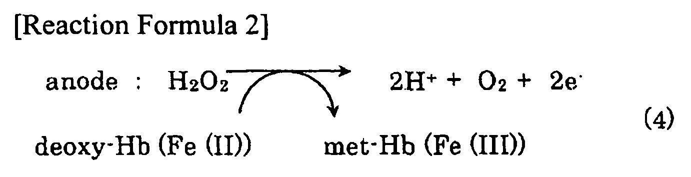

In a case where the competing reactions are generated, the hydrogen peroxide, which is supposed to be consumed in the anode 37, is consumed by the deoxyhemoglobin. Accordingly, the current value measured by the current value measuring unit 32 falls below an expected value to be fundamentally obtained. In the specimen including hemocyte, in particular, the pH in the vicinity of a hemocyte membrane tends to be lowered because gluconic acid is generated, and the oxyhemoglobin releases oxygen and is thereby transformed into the deoxyhemoglobin with accompanying the decrease of the pH. Therefore, it is considered that the production of the deoxyhemoglobin is increased in accordance with the progress of the oxidizing reaction of the glucose. Thus, the deoxyhemoglobin which inhibits the oxidizing reaction of the hydrogen peroxide in the anode 37 is generated in accordance with the progress the oxidizing reaction of the glucose (gluconic acid is generated), which is considered a probable reason why the measured oxidation current is smaller than the true value.

On the other hand, when an oxide such as an azide compound or nitrite is allowed to coexist as the suppressing agent, the oxidizing reaction of the deoxyhemoglobin (Fe (II)) into the methemoglobin (Fe (III)) is generated by the oxidative effect of the suppressing agent as illustrated in the following reaction formula (5).

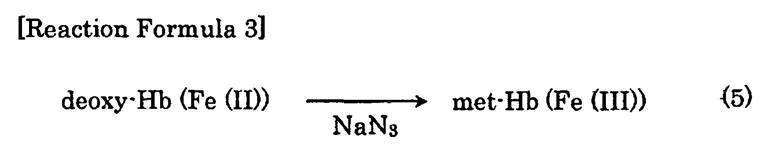

Therefore, the reaction of the hydrogen peroxide with the deoxyhemoglobin is suppressed when the suppressing agent is allowed to coexist in the specimen. As a result, the current value measured by the current value measuring unit 32 can be prevented from decreasing, and the variability of the measured response current value can be controlled in the concentration measuring device 1. Thus, the obtained result of the concentration measurement no longer shows the reduction of the value, which leads to the improvement of reproducibility, and the accuracy and reliability in the measurement can be thereby improved.

Further, ionic strength in the reaction tank 20 can be increased when the supporting electrolyte such as sodium chloride is included in the reagent, and the oxidation current obtained in the current value measuring unit 32 can be thereby stabilized. Thus, the inclusion of the supporting electrolyte can further improve the measurement reproducibility.

The present invention is not limited to the embodiment described so far, and can be variously modified. For example, the enzyme electrode may be configured to be fixated in the diluting / mixing tank, and the substrate concentration may be measured according to the batch system.

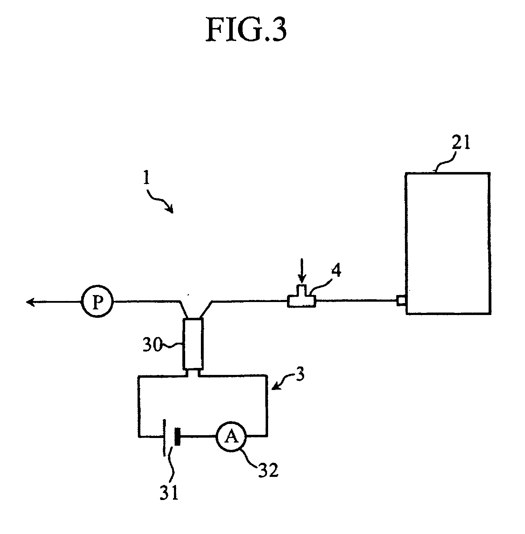

Further, the diluting / mixing tank is omitted as in the concentration measuring device 1 illustrated in Fig. 3, and a configuration may be given such that the reagent of the reagent bottle 21 is continuously supplied to the enzyme electrode 30, and the analyte and specimen are injected from an injection valve 4.

### [Example 1]

In this example, the influence of sodium azide onto a response value when a glucose concentration in a specimen is measured by means of a hydrogen peroxide electrode was discussed.

### (Specimen)

The whole blood in which the glucose concentration is adjusted to 100 mg/dL was used as the specimen.

### (Reagent)

As a reagent, a reagent 1 in which NaCl is added by 0.234 wt% to "61H" (manufactured by ARKRAY, Inc.), and a reagent 2 in which NaN₃ is further added by 0.02 wt% to the reagent 1 were respectively diluted 100 times and then supplied into a reaction tank.

### (Measurement of Response Value)

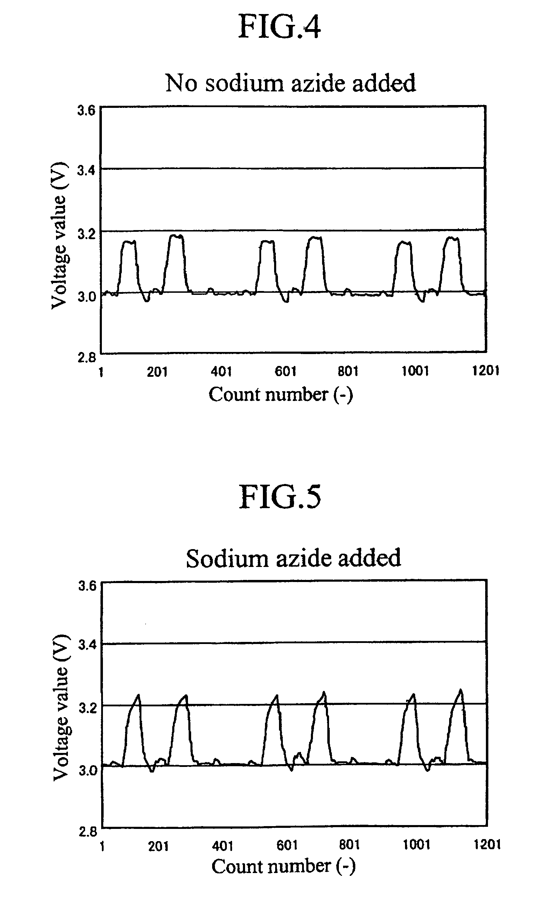

The response value was measured by ADAMS GLUCOSE GA-117 (manufactured by ARKRAY, Inc.) as an oxidation current obtained in a GOD-immobilized hydrogen peroxide electrode when the specimen and reagent were supplied into the reaction tank. The voltage to be applied to the hydrogen peroxide electrode was set to 650 mV, and a response current was measured at 50-msec intervals. An operation in which the specimen and reagent were supplied to the reaction tank and retained therein for a certain period of time and then replaced with another specimen and reagent, which were retained for a certain period of time is set as one round, and the response current was continuously measured in three rounds in total. At intervals between the rounds, the reaction tank and the pipe were washed for a certain period of time. The measurement results of response current value, which were converted into voltage values, were illustrated in the drawings, the specimen 1 in Fig. 4, while the specimen 2 in Fig. 5.

As is known when Figs. 4 and 5 are compared to each other, the specimen 1 not adding NaN₃ showed a result that maximum response values were relatively low and also variable without any distinct peak. It is considered that the low values were obtained because the amount of hydrogen peroxide measured in a hydrogen peroxide electrode was lessened when the hydrogen peroxide generated upon oxidizing glucose reacts with hemoglobin.

On the other hand, the result of the specimen 2 in which NaN₃ was added showed maximum values larger than those of the specimen 1, and a stabilized time course of the response value. More specifically, in the specimen 2 in which NaN₃ was added, it is considered that hemoglobin and NaNₐ react with each other, which is expected to prevent the amount of hydrogen peroxide measured in a hydrogen peroxide from decreasing.

It is found from these results that the measurement accuracy and reproducibility are improved when NaN₃ is added to the reaction system since the increase of the response value is observed and the maximum values thereof are stabilized.

### [Example 2]

In this example, the influences of sodium azide and sodium nitride onto a response value when a glucose concentration in a specimen is measured by means of a hydrogen peroxide electrode were discussed.

### (Specimen)

The whole blood in which the glucose concentration is adjusted to 100 mg/dL was used as the specimen.

### (Reagent)

As a reagent, a reagent 3 in which NaCl is added by 0.234 wt% to "61H" (manufactured by ARKRAY, Inc.), a reagent 4 in which NaN₃ is further added by 0.02 wt% to the reagent 3, and a reagent 5 in which sodium nitrite is added by 0.02 wt% to the reagent 3 were respectively diluted 100 times and then supplied into a reaction tank.

### (Measurement of Response Value)

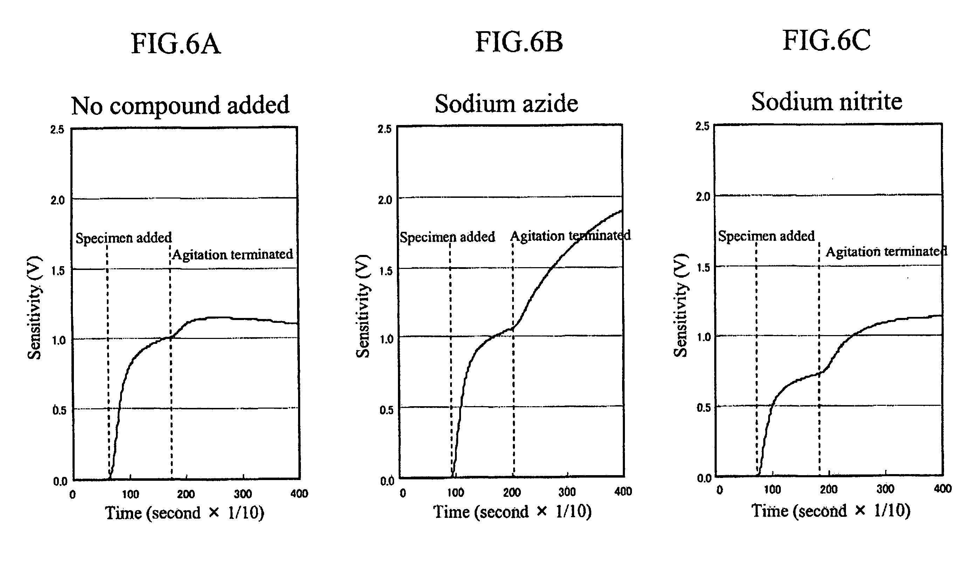

The response value was measured by ADAMS GLUCOSE GA-117 (manufactured by ARKRAY, Inc.) as an oxidation current obtained in a GOD-immobilized hydrogen peroxide electrode when the specimen and reagent were supplied into the reaction tank. The voltage to be applied to the hydrogen peroxide electrode was set to 650 mV, and a Response current was measured at 50-msec intervals. The response current was continuously measured in a sequence of operations in which the specimen and reagent were supplied into the reaction tank and agitated for a certain period of time and then the agitation was terminated. The measurement results of the response current values, which were converted into voltage values, were illustrated in the drawings, the specimen 3 in Fig. 6A, the specimen 4 in Fig. 6B, and the specimen 5 in Fig. 6C.

As is known from Fig. 6A, the specimen 3 in which NaN₃ and sodium nitrite were not added showed a deteriorated sensitivity in a while after the agitation was terminated. It is considered that it is because the oxidizing reaction of deoxyhemoglobin (oxy-Hb (Fe II)) into methemoglobin (met-Hb (Fe (III)) advances, and the anodic reaction of hydrogen peroxide is inhibited.

On the other hand, the sensitivity of the specimen 4 in which NaN₃ was added increased even after the agitation was terminated within the measurement range set in the present example as is known from Fig. 6B, while the sensitivity of the specimen 5 in which sodium nitrite was added inclined to approach asymptotically to a certain value after the agitation was terminated within the measurement range set in the present example as is known from Fig. 6A. More specifically, the deterioration of the sensitivity was not observed in a case where NaN₃ and sodium nitrite were added, which is considered that NaN₃ and sodium nitrite suppresses the oxidizing reaction of deoxyhemoglobin (oxy-Hb (Fe II)) into methemoglobin (met-Hb (Fe (III)).

### [Example 3]

In this example, the influence of NaCl in a reagent onto a response value was discussed.

As the reagent, "61H" (manufactured by ARKRAY, Inc.) was prepared as a reagent 6, and a specimen 7 in which NaCl was added to the specimen 6 by 0.234 wt% were used.

As a specimen, a physiological saline including NaCl of 0.9 wt% which was diluted 100 times with the specimen 6 or 7 was used.

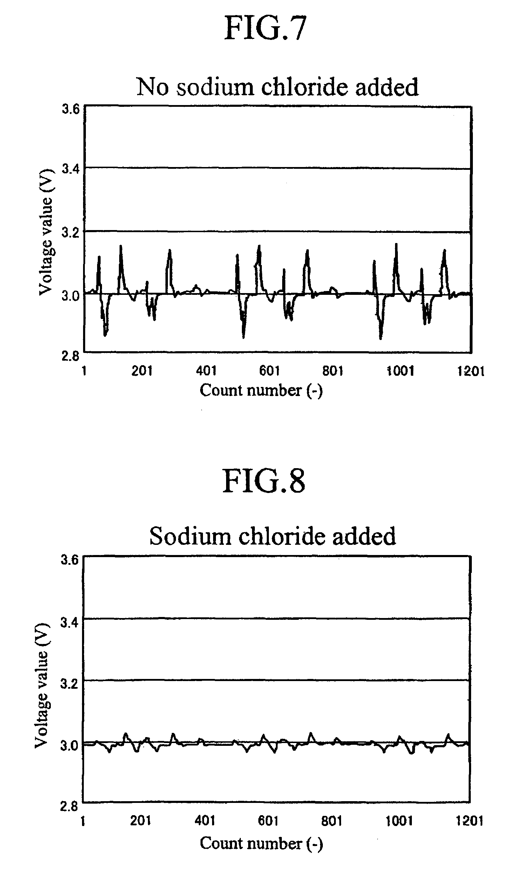

The response value was measured in a manner similar to Example 1. The measurement results of response current values, which were converted into voltage values, were illustrated in the drawings, the specimen 6 in Fig. 7, while the specimen 4 in Fig. 8.

As is known from Figs. 7 and 8, the response value was instable in the specimen 6 in which NaCl was not added, while the response value was stable in the specimen 7 in which NaCl was added. That is, it is considered that NaCl is likely to stabilize the basis of the response current value.

Therefore, it is considered that the response value is stabilized and the measurement accuracy and reproducibility are improved when NaCl is added to the reagent.

### BRIEF DESCRIPTION OF THE DRAWINGS

Fig. 1 is a sectional view schematically illustrating a structure of a concentration measuring device according to the present invention.
Fig. 2 is a sectional view schematically illustrating a structure of an enzyme electrode provided in the concentration measuring device illustrated in Fig. 1.
Fig. 3 is a pipe for describing another example of the concentration measuring device according to the present invention.
Fig. 4 is a graph showing the measurement result of a response current in the whole blood in a case where sodium azide is not added in Example 1.
Fig. 5 is a graph showing the measurement result of a response current in the whole blood in a case where sodium azide is added in Example 1.
Fig. 6A is a graph showing the measurement result of a response current value in a case where a suppressing agent is not added in Example 2.
Fig. 6B is a graph showing the measurement result of a response current in the whole blood in a case where sodium azide is added as a suppressing agent in Example 2.
Fig. 6C is a graph showing the measurement result of a response current in the whole blood in a case where sodium nitrite is added as a suppressing agent in Example 2.
Fig. 7 is a graph showing the measurement result of a response current in a physiological saline in a case where sodium nitrite is not added in Example 3.
Fig. 8 is a graph showing the measurement result of a response current in a physiological saline in a case where sodium nitrite is added in Example 3.

### DESCRIPTION OF REFERENCE SYMBOLS

- 1: concentration measuring device
- 36: hydrogen peroxide electrode

## Claims

1. A method for measuring the glucose concentration in a blood specimen containing hemoglobin and glucose, based on the amount of hydrogen peroxide generated from the glucose, comprising the steps of
contacting the blood specimen with a reagent comprising a diluent, an azide compound as a suppressing agent for suppressing the reaction between hydrogen peroxide and hemoglobin by transforming hemoglobin into methemoglobin, and sodium chloride or potassium chloride as a supporting electrolyte, wherein the concentration of the azide compound in the reagent is 0.001 to 1.000 wt%, and
measuring the concentration of hydrogen peroxide generated from the glucose with an enzyme electrode (30) comprising a substrate selective transmission film (33), an enzyme immobilized film (34), a hydrogen peroxide selective transmission film (35), and a hydrogen peroxide electrode (36), wherein the substrate selective transmission film (33) is a film for selectively supplying glucose in the specimen to the enzyme immobilized film (34), and the hydrogen peroxide selective transmission film (35) is a film for selectively supplying hydrogen peroxide to the hydrogen peroxide electrode (36).

2. The method for measuring the glucose concentration according to claim 1, wherein the suppressing agent is sodium azide.

3. The method for measuring the glucose concentration according to claim 1, wherein the concentration of the supporting electrolyte in the reagent is 0.01 to 2.00 wt%.

## Patentansprüche

1. Verfahren zur Messung der Glucosekonzentration in einer Blutprobe, die Hämoglobin und Glucose enthält, welches auf der von Glucose erzeugten Wasserstoffperoxidmenge basiert und die Schritte aufweist:
In-Kontakt-Bringen der Blutprobe mit einem Reagenz, das ein Verdünnungsmittel, eine Azidverbindung als Hemmmittel zur Hemmung der Reaktion zwischen Wasserstoffperoxid und Hämoglobin durch Umwandlung von Hämoglobin in Methämoglobin, und Natriumchlorid oder Kaliumchlorid als Grundelektrolyt umfasst, worin die Konzentration der Azidverbindung in dem Reagenz 0,001 bis 1,000 Gew.-% beträgt, und
Messung der von der Glucose erzeugten Wasserstoffperoxidkonzentration mit einer Enzymelektrode (30), die einen Substrat-selektiven Transmissionsfilm (33), einen Enzym-immobilisierten Film (34), einen Wasserstoffperoxid-selektiven Transmissionsfilm (35) und eine Wasserstoffperoxidelektrode (36) umfasst, worin der Substrat-selektive Transmissionsfilm (33) ein Film für die selektive Zuführung von Glucose in der Probe zu dem Enzym-immobilisierten Film (34) ist, und der Wasserstoffperoxid-selektive Transmissionsfilm (35) ein Film für die selektive Zuführung von Wasserstoffperoxid zu der Wasserstoffperoxidelektrode (36) ist.

2. Verfahren zur Messung der Glucosekonzentration gemäß Anspruch 1, worin das Hemmmittel Natriumazid ist.

3. Verfahren zur Messung der Glucosekonzentration gemäß Anspruch 1, worin die Konzentration des Grundelektrolyts in dem Reagenz 0,01 bis 2,00 Gew.-% beträgt.

## Revendications

1. Procédé pour mesurer la concentration de glucose dans un échantillon sanguin contenant de l'hémoglobine et du glucose, sur la base de la quantité de peroxyde d'hydrogène produite à partir du glucose, comprenant les étapes de
mise en contact de l'échantillon sanguin avec un réactif comprenant un diluant, un composé azoture comme agent suppresseur pour supprimer la réaction entre le peroxyde d'hydrogène et l'hémoglobine en transformant l'hémoglobine en méthémoglobine, et du chlorure de sodium ou du chlorure de potassium comme électrolyte de support, où la concentration du composé azoture dans le réactif est 0,001 à 1,000 % en poids, et
mesure de la concentration de peroxyde d'hydrogène formé à partir du glucose avec une électrode enzymatique (30) comprenant un film à transmission sélective pour le substrat (33), un film à enzyme immobilisée (34), un film à transmission sélective pour le peroxyde d'hydrogène (35) et une électrode à peroxyde d'hydrogène (36), où le film à transmission sélective pour le substrat (33) est un film pour fournir sélectivement le glucose dans l'échantillon au film à enzyme immobilisée (34), et le film à transmission sélective pour le peroxyde d'hydrogène (35) est un film pour fournir sélectivement du peroxyde d'hydrogène à l'électrode à peroxyde d'hydrogène (36).

2. Procédé pour mesurer la concentration de glucose selon la revendication 1 où l'agent suppresseur est l'azoture de sodium.

3. Procédé pour mesurer la concentration de glucose selon la revendication 1 où la concentration de l'électrolyte de support dans le réactif est 0,01 à 2,00 % en poids.
